Europäisches Patentamt

European Patent Office

Office européen des brevets

(19)

(11) Publication number: **0 450 221 A1**

# EUROPEAN PATENT APPLICATION

(21) Application number: 90308488.7

(22) Date of filing: 01.08.90

(51) Int. Cl.⁵: **A61M 25/06, A61M 29/00**

(30) Priority: 04.04.90 US 504610

(43) Date of publication of application:
09.10.91 Bulletin 91/41

(84) Designated Contracting States:
**BE DE ES FR GB IT NL**

(71) Applicant: **C.R. BARD, INC.**
**730 Central Avenue**
**Murray Hill New Jersey 07974(US)**

(72) Inventor: **Bourne, George W., IV**
**147 Wellman Avenue**
**North Chelmsford, Massachusetts 01863(US)**
Inventor: **Hart, John E.**
**RR1, Box 274C**
**North Berwick, Maine 03906(US)**
Inventor: **Browne, Kevin F.**
**1030 Lake Hollingsworth Drive**
**Lakeland, Florida 33803(US)**

(74) Representative: **Woodward, John Calvin et al**
**VENNER SHIPLEY & CO. 368 City Road**
**London EC1V 2QA(GB)**

(54) Expandable soft tip sheath.

(57) A catheter introducer and dilator for percutaneous placement of the introducer are provided with a configuration in which the distal end of the introducer sheath merges with the outer surface of the dilator in a manner that avoids formation of a distally facing shoulder. The arrangement provides for smooth atraumatic entry of the dilator and sheath, percutaneously, into a blood vessel. The tip configuration of the catheter introducer is such as to enable a range of catheter sizes to be used with the introducer sheath. The tip is soft to reduce risk of trauma to the inner lining of the blood vessel.

EP 0 450 221 A1

FIELD OF THE INVENTION

This invention relates to catheter introducers and dilators for percutaneous placement of the introducers.

BACKGROUND OF THE INVENTION

It is a common practice in many medical catheterization procedures to insert and remove the catheter through a previously placed catheter introducer sheath. Typically, the introducer sheath is in the form of a relatively short, somewhat flexible plastic tube that is inserted percutaneously into a body lumen, such as a blood vessel. The introducer sheath remains in place during the catheterization procedure and enables one or more catheters to be inserted in or removed from the patient's blood vessel or the like. Use of an introducer sheath reduces trauma to the patient's blood vessel and tissue in that it avoids the necessity for making repeated punctures through the patient's skin and blood vessel in order to change catheters. Once the introducer sheath has been placed it provides ready access to the body lumen into which the catheter is to be inserted. It also is useful in that it provides access to a patient's blood vessels for infusion of liquids, monitoring of blood pressure, taking samples of blood or the like. For example, U.S. Patent 4,424,833 illustrates one type of catheter introducer in which a self-sealing fitting is mounted to the proximal end of the introducer sheath.

A typical procedure for percutaneously placing a catheter introducer in a patient involves the use of a dilator, a guidewire and a hollow needle. In a typical placement arrangement, the introducer sheath is carried on a tubular dilator. The distal end (the end inserted into the patient) of the dilator is tapered. A central bore, adapted to receive a guidewire, extends through the length of the dilator and exits at the distal tip. The dilator is intended to be passed over a guidewire previously placed in the patient. The guidewire serves to guide the dilator and introducer sheath carried on the dilator percutaneously into the blood vessel or other body lumen to be accessed. It is among the objects of such systems to provide a smooth transition of the guidewire to the dilator and of the dilator to the introducer sheath so that as the dilator and introducer sheath are advanced together over the guidewire, they will enter and pass through the patient's tissues smoothly, gradually but progressively dilating those tissues. It is particularly important that the entry of the dilator and carried introducer sheath does not injure the blood vessel, as might happen, for example, if the leading end of the dilator or introducer sheath were to catch on the blood vessel. Should any portion of the blood vessel become pinched or caught by the dilator or the introducer sheath, continued advancement of the dilator and sheath could cause serious damage to the blood vessel.

Typically, the introducer sheath fits closely about the dilator. The distal end of the sheath, however, forms somewhat of a shoulder with respect to the dilator and there is some risk that the shoulder can become caught on the blood vessel as the introducer sheath advances into the blood vessel. For example, it may catch on the thin, delicate intima that lines the lumen of the blood vessel. Because of the delicate nature of the intimal lining, a physician might not realize that the lining has become caught and further advancement of the dilator and sheath likely would result in trauma to the blood vessel. Additionally, when piercing tough tissue, such as scarred or calcified tissue, the shoulder can catch on such tissue, preventing its further advancement. When continued insertion force is applied under such circumstances, the tip of the sheath may peel back on the dilator.

In addition to the foregoing, introducer sheaths are sized to be adapted to be used with a particular size catheter. The physician, however, may desire to change catheters to one having a different diameter. Catheter introducer sheaths typically in use do not lend themselves effectively to receiving a range of catheter sizes. Thus, should a physician wish to change catheters to one of a different diameter, it may be necessary also to change catheter sheaths. In such instances, the principal advantage of the catheter sheath, namely, that it enables repeated vascular, etc. access with only a single percutaneous puncture, is lost.

It also is important to minimize the risk of trauma to the inner lining of the blood vessel by the catheter introducer, even after the introducer has been placed in the blood vessel. Should the distal tip of the introducer have a hard or somewhat sharp edge that would add to the risk of injury. It would be desirable to provide a tip for a catheter introducer that is relatively soft and atraumatic.

The problem of potential injury to a blood vessel by a catheter introducer sheath becoming caught on the blood vessel during insertion has been recognized for many years. A number of devices have been proposed. Notwithstanding prior efforts to provide an atraumatic catheter introducer and dilator combination, there still remains a need for such a device. It is among the general objects of the invention to provide such a catheter introducer sheath-dilator device.

SUMMARY OF THE INVENTION

In accordance with the invention, an improved sheath and dilator combination is provided in which there is no shoulder formed at the juncture of the distal end of the sheath and the body of the dilator. The distal end of the sheath is provided with a soft, flexible tip that tapers distally to a smaller diameter than the more proximal body of the sheath. The introducer sheath is carried on a dilator in which a circumferential groove is formed about the distal region of the dilator body, just proximally of the tapered dilator tip, the tip of the dilator thus defining somewhat of an arrowhead shape. The distal tip of the dilator and the distal tip of the sheath are dimensioned so that the tapering soft tip of the sheath fits within the circumferential groove, behind the dilator head. The sheath and dilator are dimensioned such that the tip of the sheath forms a smooth surface in the region of the transition of the sheath tip to the dilator head. The arrangement provides a shoulder-free merging of the tip of the sheath with the tip of the dilator. when the dilator and sheath are advanced over the guidewire, percutaneously into the patient's blood vessel, the absence of a distally facing shoulder between the sheath and the dilator provides a smooth atraumatic entry through the skin, subcutaneous tissue and into the blood vessel. Once the introducer sheath is placed in the blood vessel, the dilator is withdrawn together with the guidewire by pulling them proximally with respect to the introducer sheath. As the dilator is withdrawn, the soft, flexible tip of the sheath deforms to permit the head portion of the dilator to pass proximally through the soft tip. The soft tip is provided with a plurality of short slits that weaken the sidewall of the soft tip and enhance the ability of the tip to stretch to accommodate the head of the dilator as well as to reduce the drag of the tip on a subsequently inserted catheter.

The ability of the soft distal tip of the introducer sheath to stretch to receive the somewhat larger diameter dilator tip also enables the introducer sheath to be used with a range of catheter diameters. Thus, the distal extremities of the soft introducer tip, beyond the slits is expandable and yieldable to allow for using a range of catheters. With the smaller of the catheters in the range, the soft tip contracts about the smaller catheter. With a larger catheter in the range , the soft tip will yield and expand diametrically to allow passage of the larger diameter catheter.

It is among the general objects of the invention to provide an improved sheath and dilator system for percutaneous insertion into a body lumen. Another object of the invention is to provide an improved sheath and dilator system that is free of a distally facing shoulder at the juncture of the distal tip of the sheath and the dilator. A further object of the invention is to provide an improved sheath and dilator arrangement in which the distal tip of the dilator is provided with a circumferential groove adapted to receive a tapered tip of the sheath.

Still another object of the invention is to provide an improved sheath and dilator arrangement which reduces the risk of trauma to the blood vessel during as well as after percutaneous insertion.

Yet another object of the invention is to provide an improved sheath and dilator system in which the sheath is provided with a soft atraumatic tip and which is adapted to receive a range of catheter sizes.

DESCRIPTION OF THE DRAWINGS

The foregoing and other objects and advantages of the invention will be appreciated more fully from the following further description thereof, with reference to the accompanying drawings wherein:

FIG. 1 is an illustration of the introducer sheath mounted on a dilator in accordance with the invention;

FIG. 2 is an enlarged cross-sectional illustration of the distal end of the sheath as seen along the line 2-2 of FIG. 3;

FIG. 3 is an enlarged end view of the sheath;

FIG. 4 is an enlarged sectional illustration of the distal end of the dilator;

FIG. 5 is an enlarged sectional illustration of the distal ends of the dilator and sheath with the dilator mounted on the sheath;

FIG. 6 is an enlarged sectional illustration of the distal end of the dilator and sheath illustrating, diagrammatically, the manner in which the soft tip of the sheath flexes during retraction of the dilator to permit the head of the dilator to be drawn through the distal tip; and

FIG. 7 is an illustration of the dilator and sheath after the dilator has been withdrawn through the tip of the sheath and with the tip of the sheath resiliently returned to its relaxed configuration.

DESCRIPTION OF THE ILLUSTRATIVE EMBODIMENT

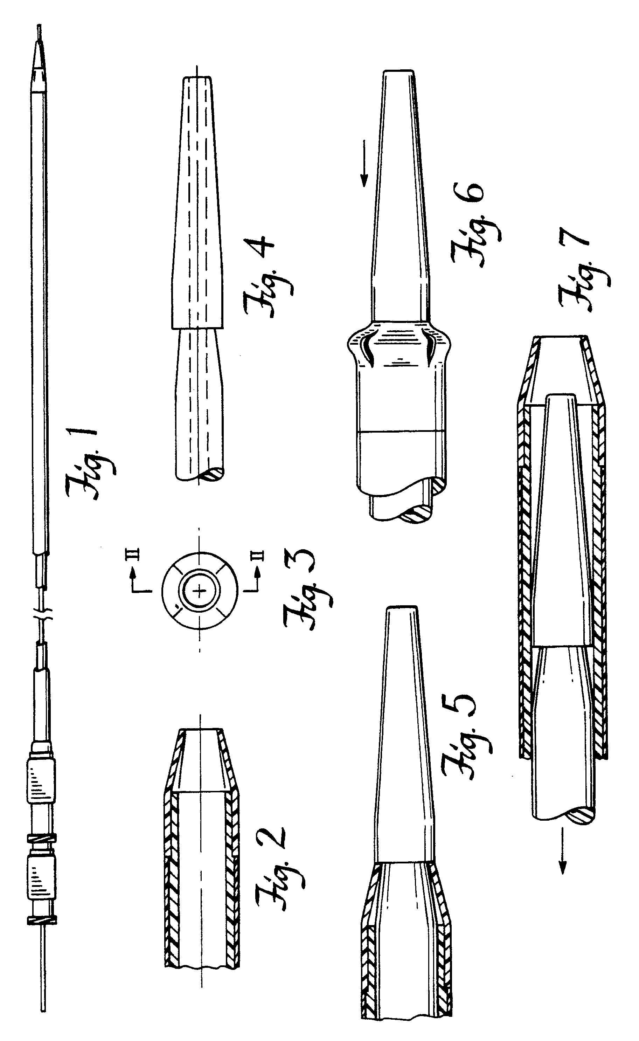

As shown in FIG. 1, the present invention includes a dilator, indicated generally by the reference character 10, and an introducer sheath 12 that fits closely over and is carried by the dilator. Both the dilator 10 and sheath 12 may be formed from any of a variety of polymeric, biocompatible materials, the dilator being formed from a more rigid polymer than the sheath 12. By way of example, the dilator may be formed from a suitable medium to high density polyethylene. The sheath may be formed from a polyurethane-nylon blend sold under the trade designation PEBAX available from Atochem Corp., Glenrock, New Jersey. The sheath preferably is formed in two coextruded layers. The inner layer 13 is extruded from PEBAX filled with bismuth subcarbonate for radiopacity and may have a hardness of the order of 63 D Shore. By way of example, for an 8 French sheath, the wall thickness of the inner layer may be of the order of 0.006" thick. The outer, coextruded layer 15 is formed from a softer PEBAX, of the order of 50 D Shore, and is unfilled. The outer layer may be of the order of 0.002" thickness for an 8 French sheath. The coextruded outer layer provides enhanced kink resistance for the sheath. Additionally, it is desirable to coat the inner and outer surfaces of the sheath with a suitable film to enhance the lubricity of those surfaces. For that purpose, it is preferred to coat the inner and outer surfaces of the PEBAX sheath with a hydrophilic coating such as polyethylene oxide.

Each of the dilator 10 and sheath 12 is provided with a fitting 14, 16, respectively, attached to its proximal end. The fitting for the dilator 10 may include a single luer fitting 14. The fitting 16 for the introducer sheath may include luer tapers but preferably also includes a hollow housing with an opening 18 at its proximal end. The housing may incorporate a self-sealing valving element (not shown), for example, of the type disclosed in U.S. Patent 4,424,833 the disclosure of which is incorporated herein by reference. The fitting 16 preferably also has a side port 20 through which liquids may be infused or blood samples taken.

The dilator 10 is formed to include a central lumen 22 which extends from the proximal fitting 14 to an outlet opening 24 at the distal extremity of the dilator 10. The distal tip of the dilator is tapered, as indicated at 26. The dilator 10 also is formed to include a circumferential tapering groove 28 just proximal to the tapered head 27. The circumferential groove 28 may be considered as being defined by a distally tapering section 30 which, at its distal end, meets an annular, proximally facing shoulder 32. Thus, it may be appreciated that the distal tip of the dilator defines somewhat of a tapering head 27. By way of illustrative example, the dilator may be about 15 cm long, about 0.115" diameter, having a lumen about 0.052" diameter. The groove 28 may be of the order of 0.015" deep at the shoulder 32. The tapered head may be about 1.0 cm long.

The catheter introducer sheath 12 is formed from an elongate tubular body 34 that is shorter than the dilator 10. By way of example, an approximately 8 French introducer sheath may be of the order of 0.136" outer diameter and 0.115" inner diameter so that it fits snugly, but slidably on the dilator 10.

In accordance with the invention, the distal end of the introducer sheath 12 is provided with a soft tip, indicated generally at 36, formed from a softer polymeric material than that from which the sheath body 34 is formed. The soft tip 36 also is formed from PEBAX having a lower durometer, preferably of the order of 30 Shore D, than the sheath. In the illustrative embodiment, the soft tip 36 includes a proximal cylindrical portion 38 which may be attached by heat bonding, such as by a lap joint, to the distal end of the sheath body 34 about a reduced diameter segment 40 at the distal end of the sheath body 34. It is preferred that for the illustrative embodiment of the invention, the overlapping cylindrical portion 38 of the tip 36 is about 1-2 mm long and the distally extending portion of the soft tip 36 is about 2-3 mm long. The tip 36 and distal segment 40 are dimensioned so that the outer surface of the proximal portion 38 of the soft tip 36 will merge smoothly with the outer surface of the sheath body 34. The soft tip 36 also includes a distal segment 42 that extends distally beyond the distal end of the distal segment 40 of the sheath body 34. The distal segment 42 of the soft tip 36 tapers inwardly in a distal direction and terminates at a distal opening 44. The inner diameter defined at the distal opening 44 preferably, for the 8 French introducer sheath of the example, is 0.080". In general, it is desired for the sheath of the present invention to form a distal tip opening 44 so that its inner diameter is 2 to 2.5 French sizes (0.026" to 0.033") less than the inner diameter of the sheath body 34.

The soft tip 36 is configured so that when the introducer sheath 12 is mounted on the dilator 10, the tapering distal segment 42 will fit within the circumferential groove 28 on the dilator 10 with the end of the distal segment 42, at the opening 44, butting against the shoulder 32 formed on the dilator tip. Preferably, the dilator 10 and sheath 12 are of a length such that when the dilator and sheath fittings 14, 16 are mated, the distal segment 42 of the soft tip will be disposed within the circumferential groove 28 of the dilator 10. When the sheath 12 is so mounted on the dilator 10, the outer surface of the distal segment 42 of the soft tip merges smoothly with or is slightly below the outer surface of the tapered head portion 27 on the dilator to provide a smooth transition from the dilator head 27 to the tip of the introducer sheath. when the dilator and sheath are in this assembled configuration and are advanced in unison over a guidewire, percutaneously and into a blood vessel, there is no distally facing shoulder at the juncture of the distal end of the sheath and dilator that might catch on and injure the blood vessel.

As shown in FIGS. 1 and 3, the distal segment 42 of the soft tip 36 is weakened, preferably by a plurality of longitudinally extending slits 46. The slits terminate short of the opening 44 at the outer end of the distal segment 42 so that the distal tip of the distal segment 42 defines an uninterrupted circumferential band about the distal opening 44. The slits 46 are intended to weaken the sidewall of the distal segment to present less resistance to dilation of the distal segment 42, as when a relatively large diameter catheter is passed through the introducer, as will be described.

FIGS. 6 and 7 illustrate, somewhat diagrammatically, the manner in which the soft tip 36 yields as the dilator 10 is withdrawn from the introducer sheath 12. As shown in FIG. 6, when the dilator is withdrawn the proximally facing shoulder 32 of the dilator pushes the distal segment 42 of the soft tip 36 proximally. The longitudinal slits 46, which may be considered as defining a number of longitudinally extending segments 45, serve to weaken the distal segment 42 and cause it to fold in a manner that the longitudinally extending segment 45 buckle as shown in FIG. 6. The distal segment 42 of the soft tip 36 continues to collapse until the shoulder 32 of the dilator 10 finally is pulled through the distal opening 44, the uninterrupted circumferential band that defines the distal opening 44 stretching and yielding to permit passage of the slightly larger diameter shoulder 32. Once the shoulder 32 has been drawn through the opening 44, the resilient distal segment 42 returns to its extended configuration while the dilator continues to be withdrawn, as suggested in FIG. 7.

As will be appreciated by those familiar with the use of catheter introducers, the catheter introducer is placed by a procedure known as the Seldinger technique in which a hollow needle first is inserted percutaneously into a blood vessel to be catheterised. After the needle has punctured and entered the blood vessel, a guidewire is passed through the needle and into the blood vessel. With the guidewire in place in the blood vessel, the needle is withdrawn over the guidewire. The combined dilator and catheter introducer sheath then are advanced over the guidewire, percutaneously into the blood vessel. It will be appreciated that the smooth unshouldered transition between the head 27 of the dilator and the distal tip 36 of the introducer sheath provide a progressively and smoothly increasing diameter to dilate the entry into the blood vessel but in a shoulder-free manner that reduces the risk of trauma and injury to the blood vessel. Once the catheter introducer has been so placed in the blood vessel, the dilator and guidewire are removed. The introducer sheath then is in readiness to receive and guide catheters into the blood vessel.

Also among the advantages of the invention is that the catheter introducer is adapted to accept a range of catheter sizes. For example, a catheter having a sheath body 34 of an 8 French size (0.104") may have a soft tip 36 in which the distal segment 42 tapers to define a 6 French (0.078") distal opening 44. Such a catheter introducer is adapted to receive catheters in the range of 6 to 8 French. With a 6 French catheter, the catheter will be closely engaged by the circumferential band that defines the distal opening 44. Should it be desired to insert a size 7 or 8 French catheter into the patient, the introducer sheath also is adapted to receive that. Such a larger size catheter, inserted through the introducer sheath, will cause the circumferential band at the distal tip of the distal segment 42 to dilate resiliently, thus accommodating the larger diameter catheter. Additionally, the soft, flexible tip also serves to reduce the risk of injury to the delicate lining of the blood vessel lumen after the introducer 12 has been placed.

From the foregoing, it will be appreciated that the invention provides a catheter introducer and associated dilator in which the juncture of the distal end of the catheter introducer and the dilator define a smooth, shoulderless transition that avoids injury or trauma to the blood vessel during percutaneous insertion. Additionally, the introducer is adapted to receive a range of catheters. Moreover, the catheter introducer sheath is provided with a soft, flexible and deformable tip which, when disposed within the blood vessel, will reduce the risk of trauma to the inner surface of the blood vessel.

It should be understood, however, that the foregoing description of the invention is intended merely to be illustrative thereof, and that other modifications, embodiments and equivalents may be apparent to those skilled in the art without departing from its principles.

Having thus described the invention, what we desire to claim and secure by Letters Patent is:

## Claims

1. A catheter introducer assembly comprising:

an elongate tubular dilator having a body, a guidewire lumen extending through the body and a tapered head at the distal end of the body, the body being formed to define a circumferential groove proximally of the tapered head; and

an elongate, flexible catheter introducer sheath mounted on the body of the dilator, the sheath having a soft resilient tip, a distal segment of which is tapered to a smaller diameter than the proximal portion of the sheath body, the soft tip being tapered and dimensioned such that when the sheath is mounted on the dilator the distal portion of the soft tip will be received in the circumferential groove, thereby to minimize trauma to tissue upon insertion of the assembly through tissue.

2. A catheter introducer assembly as defined in claim 1 wherein the outer surface of the soft tip and the outer surface of the tapered head of the dilator define a smooth, unshouldered transition surface.

3. A catheter introducer assembly as defined in claim 1 wherein the distal segment of the soft tip is weakened.

4. A catheter introducer assembly as defined in claim 3 wherein the distal segment of the soft tip is weakened by a plurality of longitudinal slits formed in the distal segment of the soft tip.

5. A catheter introducer assembly as defined in claim 4 wherein the longitudinal slits terminate short of the distal end of the distal segment thereby to define an uninterrupted circumferential band about the distal tip of the soft tip.

6. A catheter introducer comprising:
   an elongate flexible tube having a proximal end and a distal end;
   a soft tubular tip attached to the distal end of the tube and having a distal segment that extends distally of the distal end of the tube, the tip being formed from a material that is softer and more flexible than the material from which the tube is formed;
   the distal segment of the tip being tapered to define a distal opening that is smaller in diameter than the inner diameter of the tube.

7. A catheter introducer as defined in claim 6 wherein the distal segment is disrupted in a manner so as to weaken the distal segment.

8. A catheter introducer as defined in claim 6 wherein the distal segment of the soft tip is weakened by a plurality of longitudinal slits formed in the distal segment, the longitudinal slits terminating distally short of the distal end of the distal segment, thereby to define an uninterrupted circumferential band about the distal tip of the distal segment.

9. A catheter introducer assembly as defined in any of claims 1-5 wherein the sheath is coextruded and includes an inner relatively thick layer and an outer relatively thin layer, the outer layer being formed from a softer material than the inner layer.

10. A catheter introducer as defined in any of claims 6-8 wherein the sheath is coextruded and includes an inner relatively thick layer and an outer relatively thin layer, the outer layer being formed from a softer material than the inner layer.

11. A catheter introducer assembly as defined in any of claims 1-5 wherein the inner surface of the sheath is coated with a hydrophilic material.

12. A catheter introducer assembly as defined in any of claims 6-8 wherein the inner surface of the catheter introducer is formed from a hydrophilic material.

13. A catheter introducer assembly as defined in any of claims 1-5 wherein the introducer sheath is coextruded to include an inner layer and an outer layer, the outer layer being thinner and formed from a softer material than the inner layer.

14. A catheter introducer as defined in any of claims 6-8 wherein the introducer sheath is coextruded to include an inner layer and an outer layer, the outer layer being thinner and formed from a softer material than the inner layer.

15. A catheter introducer assembly as defined in claim 1 wherein the orifice at the tip of the sheath is approximately 2 to 2.5 French sizes smaller than the inner diameter of the sheath.

16. A catheter introducer as defined in claim 6 wherein the orifice at the tip of the sheath is approximately 2 to 2.5 French sizes smaller than the inner diameter of the sheath.

Fig. 1

Fig. 2

Fig. 3

Fig. 4

Fig. 5

Fig. 6

Fig. 7

## DOCUMENTS CONSIDERED TO BE RELEVANT

EP 90308488.7

| Category | Citation of document with indication, where appropriate, of relevant passages | Relevant to claim | CLASSIFICATION OF THE APPLICATION (Int. Cl.5) |
|---|---|---|---|
| X | <u>WO - A2 - 90/02 579</u><br>(MALLINCKRODT INC.)<br>  * Fig. 1,2,11; abstract; page 6, line 16 - page 3, line 23; page 9, line 23 - page 10, line 12; page 12, lines 4-12 * | 6,12, 16 | A 61 M 25/06<br>A 61 M 29/00 |
| A | | 1 | |
| A | <u>US - A - 4 909 798</u><br>(J.J. FLEISCHHACKER et al.)<br>  * Fig. 1; column 5, lines 30-58 * | 1,6 | |
| A | <u>US - A - 4 850 975</u><br>(Y. FURUKAWA)<br>  * Totality; especially column 4, line 18 - column 5, line 30 * | 1 | |

TECHNICAL FIELDS
SEARCHED (Int. Cl.5)

A 61 M

The present search report has been drawn up for all claims

| Place of search | Date of completion of the search | Examiner |
|---|---|---|
| VIENNA | 11-07-1991 | LUDWIG |